# EUROPEAN PATENT APPLICATION

(11) **EP 2 261 379 A1**
(43) Date of publication of application: **15.12.2010**
(21) Application number: 09729455.7
(22) Date of filing: 08.04.2009
(51) Int. Cl.: C12Q 1/70, G01N 33/569, G01N 21/65

(54) **METHOD AND SYSTEM FOR DETECTING AND/OR QUANTIFYING BACTERIOPHAGES, USE OF A MICROELECTRONIC SENSOR DEVICE FOR DETECTING SAID BACTERIOPHAGES AND MICROELECTRONIC SENSOR DEVICE FOR IMPLEMENTING SAID METHOD**

(30) Priority: 09.04.2008 ES 200801007
(71) Applicant: Consejo Superior De Investigaciones Científicas, 28006 Madrid (ES); Universidad de Barcelona, 08007 Barcelona (ES)
(72) Inventor: GARCÍA ALJARO, Cristina, E-08193 Bellaterra (Barcelona) (ES); MUÑOZ BERBEL, Xavier, E-08193 Bellaterra (Barcelona) (ES); MUÑOZ PASCUAL, Francisco Javier, E-08193 Bellaterra (Barcelona) (ES); BLANCH GISBERT, Anicet R., E-08007 Barcelona (ES)
(74) Representative: Pons Ariño, Angel
(86) International application number: PCT/ES2009/070095
(87) International publication number: WO 2009/125043

(57) **Abstract**

The present invention relates to a method and system for detecting and/or quantifying bacteriophages capable of infecting a predetermined bacterial host strain, which is based on the technique of plasmon surface resonance that takes place on the conductive-material surface of an optical sensor device, and also relates to a microelectronic sensor device for implementing said method and to the use of said microelectronic sensor device for detecting and/or quantifying said bacteriophages

## Description

The present invention relates to a process and system for detecting and/or quantifying bacteriophages capable of infecting a predetermined bacterial host strain that is based on the surface plasmon resonance technique that takes place on the conductive material surface of an optical sensor device.

It also relates to a microelectronic sensor device for carrying out this process and to the use of this microelectronic sensor device for detecting and/or quantifying bacteriophages.

### BACKGROUND OF THE INVENTION

Bacteriophages are viruses that infect bacteria. They are composed of nucleic acids (DNA or RNA) enclosed in a protein capsid. These viruses have a high specificity for the bacteria that they infect so that a specific bacteriophage or group of bacteriophages can be detected by the selected bacterial host strain.

In summary, the bacteriophage cycle consists of 4 phases: in the first step, the bacteriophage- bacterium recognition occurs as does the injection of the phage nucleic acids, which contain the genetic information necessary for the formation of new bacteriophages. Next, the bacteriophage genetic material integrates into the bacterial chromosome in the form of DNA. Once integrated, the infection of the host cell can result in two types of response depending on the type of bacteriophage that infects it. Thus, the temperate bacteriophages, which may be integrated in the genome of the host cell indefinitely or until lysis has occurred and the lytic bacteriophages, which after a period of incubation and using the biosynthetic machinery of the host cell, replicate the material necessary for the creation of new virus particles and are released by lysis of the host cell, are distinguished.

The detection and/or quantification of bacteriophages of enteric bacteria is of particular importance in the microbiological control of waters and in the determination of the origin of faecal contamination. These bacteriophages have been proposed as microorganism indicators of the presence of viruses as they have a similar behaviour to viruses. There are three groups of bacteriophages of enteric bacteria that are used as indicators of faecal contamination of virus origin: somatic coliphages, F-Specific RNA phages and phages that infect *Bacteriodes* species.

Another group of bacteriophages where detection and/or quantification is of particular interest is that of the bacteriophages infecting lactic bacteria of the type that are used for the production of cheeses or other fermented dairy products. These bacteriophages seriously affect the production of lactic acid and result in large financial losses for the dairy fermentation industries.

The classical methods for the detection of bacteriophages are based on the detection of their effects on host bacteria. Traditionally, for counting bacteriophages, an assay using a double agar layer is used. One layer of semi-solid agar is deposited containing a specific host strain (depending on the bacteriophage to be quantified) and the sample to be analysed is added on an agar layer. The bacteriophages are quantified by the detection of zones or plaques of lysis caused by the infection and lysis of the host bacteria by a bacteriophage or groups of bacteriophages present in the sample, after a period of incubation of this host strain.

The methods based on seeding on plates have the disadvantage of being very slow, requiring at least 24 hour of incubation.

Other more recently described methods for the detection of bacteriophages are based on the detection of these bacteriophages by the PCR technique. However, these methods are expensive and in addition very laborious.

European patents EP0149383 and EP0714450 disclose other methods for detecting bacteriophages, specifically for detecting lactic bacteria bacteriophages.

Patent EP0714450 relates to the detection of an internal bacterial component after lysis using emission of bioluminescence in enzymatic reactions catalysed by enzymes such as luciferase. Patent EP0149383 relates to the detection of the inhibition of growth by the use of pH indicators for detecting microbial activity.

The methods described in the cited patents have the drawback that they do not allow the detection of bacteriophages in their active state, so that in practice they are not very reliable.

These are also very complex and expensive methods as they involve the use of a large number of reagents.

### DESCRIPTION OF THE INVENTION

A first objective of the present invention is that of developing an alternative process and system for detecting and/or quantifying bacteriophages that is based on the use of an optical sensor device that uses the Superficial Plasmon Resonance technique, hereafter referred to as the SPR technique.

In accordance with this objective, according to a first aspect, the present invention provides a process for detecting and/or quantifying bacteriophages capable of infecting a predetermined bacterial host strain, which comprises the stages of:
a) attaching bacteria from at least one of said host strains to the conductive material surface in a microelectronic optical sensor device based on the surface plasmon resonance technique,
b) exposing the bacteria attached to this surface to a solution of material to be analysed that may contain bacteriophages,
c) incubating the solution from stage b) in predetermined conditions with the aim that, if the bacteria attached to the conductive material surface of the sensor are infected by bacteriophages, these bacteria will be lysed,
d) measuring the resonance angle of incident light on the sensor while carrying out the incubation of stage c) with the aim of detecting the change in the resonance angle caused by bacterial lysis.

With the same objective and according to a second aspect, the present invention provides a system for detecting and/or quantifying bacteriophages capable of infecting a predetermined bacterial host strain that comprises a microelectronic optical sensor device based on the surface plasmon resonance technique, bacteria of the host strain attached to the conductive material surface of this device and means of processing and control for detecting the change in the resonance angle of light incident on the sensor, this change being caused by the lysis effect when the bacteria are infected and lysed by the bacteriophages.

A second objective of the present invention is that of providing a microelectronic optical sensor device based on the surface plasmon resonance technique that comprises bacteria attached to the conductive material surface of the sensor, wherein these bacteria belong to at least one predetermined host strain, selected to be infected by bacteriophages of the specificity predetermined by the strain.

Lastly, a third objective of the present invention is the use of a microelectronic optical sensor device for detecting and/or quantifying bacteriophages capable of infecting a predetermined bacterial host strain by the surface plasmon resonance technique.

In the present invention, the term microelectronic device is understood to mean a flat sensor component, preferably manufactured by thin layer microelectronic technologies.

The term conductive material is understood to mean any conductive material, preferably metal, but also doped conductive oxides.

Similarly, surface plasmon resonance is understood to mean the technique based on the detection of the change in the refractive index that takes place in a solution placed close to the conductive material surface of an optical sensor device.

The phenomenon of surface plasmon resonance occurs when a light beam illuminates the interface between two media with different refractive indexes, between which a fine layer of conductive material, for example a metal, has been inserted. The evanescent wave created by total internal reflection interacts with the oscillating electrons or plasmons of the metal, causing a reduction in the intensity of the reflected light. The resonance angle of this phenomenon is sensitive to the refractive index of the solution next to the metal or conductive material. As the wavelength of the incident light and the refractive index of the metal and of the substrate whereon the metal is deposited remain constant, the resonance angle only varies with changes in the refractive index of the solution next to the metal. For this reason and based on this phenomenon, macromolecular interactions that take place in the solution next to the metal or conductive material can be monitored by changes in the resonance angle.

Currently, optical sensors based on the SPR technique are used for detecting a large variety of biomolecular interactions such as, for example, antigen-antibody interactions. However, the use of these optical sensors for detection of phage activity has not been described.

The present invention provides a process and system for detecting bacteriophages that uses an optical sensor device based on the SPR technique.

The process and system claimed has the advantage that it is very simple, reliable and highly sensitive.

As described above, the process and system claimed is based on the detection of the change in the refractive index or change in the resonance angle that takes place in a solution, which may contain bacteriophages, placed next to the conductive material surface of an optical sensor device whereto bacteria have been attached.

The experiments performed allowed the observation that the change in the resonance angle of light incident on the optical sensor has its origin in the phage activity of the bacteriophages on the bacteria attached to the conductive material surface of the sensor.

Specifically, it was observed that phage replication of the bacteriophages generates an increase in the resonance angle that is due to bacterial lysis and to the adsorption of the remains of bacterial proteins and remains of phage particles to the conductive material surface of the sensor device. The reading of the change in resonance angle that is obtained by the SPR technique enables the detection and quantification of bacteriophages in the solution of material to be analysed.

According to a preferred embodiment, the bacteriophage concentration is determined according to the time elapsed before detecting the change in the resonance angle using the corresponding calibration curve that correlates the detection time with the bacteriophage concentration.

The system and process of the present invention has the advantage that it is highly reliable, given that it detects bacteriophages in an active state. In addition, this is a very sensitive and quick method that enables detection of 10² PFU/ml (Plaque Forming Units) in less than 2 hours. This is also a very simple method and system, easily reproducible and that can be automated, which allows monitoring the bacteriophage concentration in a sample in real time. It also can be applied to the detection of bacteriophages present in any type of matrix (waste waters, purification plant sludges, milk, bio-solids, etc.).

As described above, both the claimed system and process require the attachment to the surface of the sensor device of the host strain for the bacteriophage or group of bacteriophages to be detected. This bacterial attachment can be carried out by various techniques.

According to one embodiment, attachment can be carried out by prior adsorption to the conductive material surface of the sensor of a chemical or biological material for immobilising bacteria.

The adsorption is preferably carried out by using biological material that includes a solution of avidin or a protein derived from avidin. This solution is designed to bind bacteria that have previously been biotinylated. It has been observed that immobilisation based on the interaction between avidin and biotin retains biomolecular activity on the surface of the sensor in a more effective way than other methods.

Advantageously, said protein derived from avidin is preferably CaptAvidin.

CaptAvidin has the advantage that it enables a reversible interaction with the biotinylated bacteria so that it allows the sensor surface to be regenerated for repeat use. It has also been observed that immobilisation based on the interaction between CaptAvidin and biotin improves the sensitivity of the claimed detection process very significantly.

According to another embodiment, this prior adsorption is carried out by means of antibodies capable of recognising the bacteria to be immobilised.

According to another embodiment, attachment is carried out by the formation of self-assembled monolayers (SAM) on the conductive material surface of the sensor whereon the avidin is immobilised in a specific way. Thiolated compounds with epoxy or aldehyde functional groups are preferably used for the formation of self-assembled monolayers.

In any case, the present invention provides a microelectronic sensor device for detecting bacteriophages that constitutes in itself a valuable diagnostic tool that allows monitoring bacterial lysis in real time without the need to use labelled reagents.

The microelectronic device is also preferably a sensor chip or flat miniaturised microelectronic device, preferably manufactured using thin layer lithographic technologies. The chip is a very sensitive sensor, which enables taking measurements in very small sample volumes and in very short times owing to its small size.

The conductive material of this microelectronic device is also preferably gold. However, as described above, other metals, such as for example silver, can also be used.

The process and system claimed can be applied to the detection of any type of bacteriophages, provided that the host strain for this bacteriophage with the predetermined specificity is available. The bacteriophages can come from samples of very diverse materials; for example waters, water purification sludges, milk or other types of solid or liquid materials for which the detection of bacteriophages is industrially interesting.

In the specific case of samples from waters, the detection of bacteriophages is especially interesting because these have been proposed as microorganism indicators of the presence of viral faecal contamination.

According to a preferred embodiment, the present invention relates to the detection of bacteriophages of importance in the microbiological control of waters and/or in the determination of faecal contamination, among which are included, for example, somatic coliphages, specific F-RNA bacteriophages and bacteriophages that infect *Bacteroides fragilis.*

According to other embodiments, bacteriophages of the present invention are bacteriophages that infect lactic bacteria, such as for example:
*Lactobacillus bulgaricus, Lactobacillus lactis, Lactobacillus helveticus, Lactobacillus plantarum, and Streptococcus thermophilus.*

### BRIEF DESCRIPTION OF THE FIGURES

For better understanding of what has been described above, figures are attached by way of a non-limiting example, wherein a practical case of embodiment for the detection and quantification of somatic coliphages present in samples from waste waters is schematically represented.

In these figures:
Figure 1 is a schematic view of the structure of a microelectronic sensor device, specifically an optical sensor chip, used for detecting bacteriophages by the surface plasmon resonance technique.
Figure 2 shows a graphical representation over time of measurements of the resonance angle taken in a control sample and in a sample of waste water containing the ΦX174 somatic coliphage capable of infecting the bacterial strain of *Escherichia coli* ATCC 700078.
Figure 3 shows a graphical representation over time of normalised measurements of the resonance angle taken in a series of solutions containing different concentrations of the ΦX174 somatic coliphage. The measurements shown are those taken during the incubation phase of the solutions, wherein the increase in the resonance angle due to lysis occurs.
Figure 4 is a graphical representation showing the normalised measurements of the resonance angle of three samples of reference waste water analysed, GA2007, HM1402 and HM2100. The measurements shown are those taken during the incubation phase of the samples, in which the increase in the resonance angle due to lysis occurs.

### DESCRIPTION OF A PREFERRED EMBODIMENT

Below, an example is described of an embodiment of the process and system of the present invention for detecting the ΦX174 bacteriophage, also known as the WG5 strain according to the international ISO 10705-2 standard, which infects the host strain of *Escherichia coli* ATCC 700078.

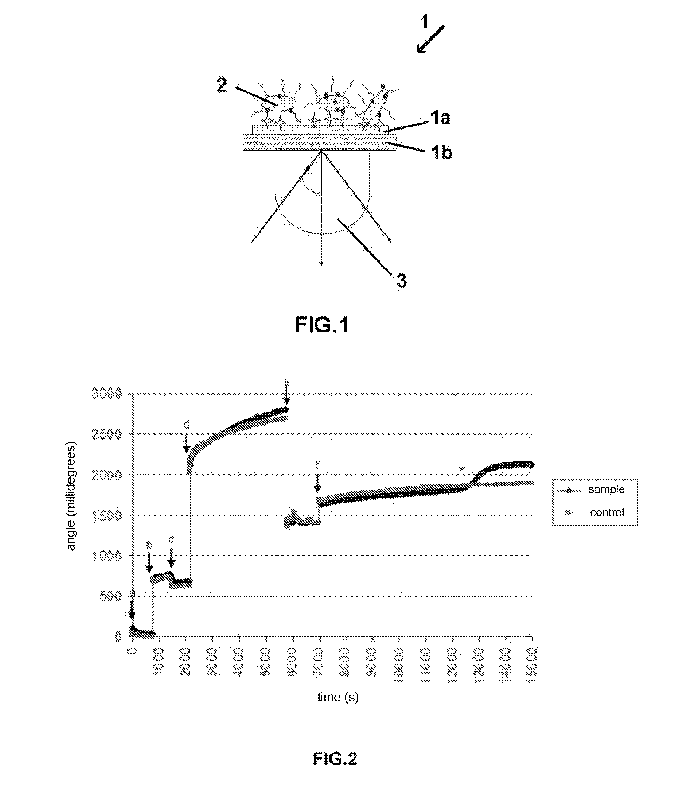

In the embodiment described, the microelectronic device used is an optical sensor chip 1, as shown in Figure 1, comprising a 50 nm layer 1 a of gold deposited on a silicon substrate 1 b.

As was described in the detailed description of the invention, the claimed process and system are based on the surface plasmon resonance phenomenon that takes place on the sensor chip 1, whereto bacteria 2 have previously been attached of a predetermined host strain for the bacteriophage or group of bacteriophages that are to be detected.

In the embodiment described, the bacteria 2 are attached to the gold surface 1a of the chip 1 by means of avidin-biotin bonds. To achieve this, a functionalisation was performed of the gold surface of the chip 1 by direct adsorption of 50 µl of an avidin solution (0.5 ml/ml) for 10 minutes. Then, the surface 1a of the chip 1 is washed with phosphate buffer and bacteria 2, previously biotinylated according to standard processes, are immobilised.

To carry out the immobilisation of the bacterial strain of *Escherichia coli* ATCC 700078 to the active surface of the chip 1, the biotinylated bacteria 2 is incubated for 1 hour at 37 °C in phosphate buffer. The incubation enables the binding of the bacteria 2 to the absorbed avidin and therefore the attachment of these bacteria 2 by avidin-biotin bonds to the surface 1 a of the chip 1.

Once the bacteria 2 are attached to the surface 1 a of the chip, the detection of bacteriophages is carried out. To do this, the chip 1, specifically the metal surface 1a whereon the bacteria 2 are attached, is exposed to a bacteriophage enrichment culture medium, wherein a sample of the solution of material to be analysed has been previously inoculated. Next, the solution in contact with the chip 1 is incubated until the resonance signal is detected.

Once the whole detection process has been performed, the used chip 1 is washed with ethanol and water and is subjected to thirty minutes ozonation treatment to allow it to be reused.

In the example of embodiment described, three different samples of waste water of urban origin containing different concentrations of ΦX174 somatic coliphage bacteriophages were analysed. To prepare the samples, 10 µl of the corresponding sample were incubated in 90 µl of MSB culture medium in each case for enrichment of the somatic coliphages. The MSB culture medium used for the enrichment of somatic coliphages was prepared as described in the international standard ISO 10705-2.

The processing of the samples, before inoculation, consisted of a centrifugation for 10 minutes at 1000 rpm and a filtration in a 0.22 µm low protein adsorption filter.

The measurements of the resonance angle were made while the solution of material to be analysed was incubated at 37 °C in contact with the chip 1 so that if the bacteria 2 were infected by bacteriophages, the change in the resonance angle caused by bacterial lysis would be detected.

The reading of the change in the resonance angle caused by this lysis is carried out using commercial SPR equipment, which analyses the intensity of light reflected by the optical sensor 1 by means of a diode detector.

For the reading of the resonance angle, the sensor chip 1, whereon the resonance phenomenon takes place, is placed in contact with a coupling prism 3 in an optical system wherethrough the polarised light is focussed towards the non-modified plane of the sensor chip 1, in this case the silicon plane 1 b. The polarised incident light is coupled to the electron field through this prism 3 to create an energy wave that extends through the dielectric material deposited on the surface 1a of the chip 1 (in the embodiment described, this was the MSB culture medium inoculated with the solution of material to be analysed). The interaction of the evanescent wave with the oscillating electrons or plasmons of the conductive material causes a reduction in the intensity of reflected light that is detected and analysed by the commercial SPR equipment. The resonance angle given by the phenomenon described is sensitive to the refractive index of the solution next to the metal 1a, so that changes in the refractive index of this solution can be monitored by reading the resonance angle.

In the present invention, the experiments performed made it possible to observe that phage replication of somatic coliphages present in the solution of material to be analysed modified the refractive index of the solution next to the metal 1 a of the sensor 1, which translates to an increase in the resonance angle of the incident light. This increase was attributed to bacterial lysis and the adsorption of the remains of bacterial proteins and remains of phage particles to the conductive material surface 1a of the sensor 1.

Figure 2 shows a graphical representation over time of the measurements of the resonance angle taken in a control sample and in one of the samples of waste water analysed that contained ΦX174 somatic coliphages. The graphic representation includes measurements of the angle during the functionalisation phase of the chip 1, the bacterial immobilisation phase and the detection phase of the bacteriophages, wherein the solution of material to be analysed was incubated in contact with the chip 1.

As can be seen in Figure 2, the sample containing bacteriophages shows an increase in the resonance angle of around 150 millidegrees in a detection time no greater than two hours (the point of increase in the reading of the resonance angle is marked with an asterisk). This finding is attributed to the activity of the bacteriophages.

To calibrate the sensor 1, the quantity of bacteriophages detected by a traditional double agar layer assay, described in ISO 10705-2, and their detection by the method of the invention using the SPR technique, were compared in parallel.

For this, a stock of the ΦX174 bacteriophage of 10¹⁰ PFU/ml (Plaque Forming Units) was prepared and decimal dilutions were performed in phosphate buffer, which were evaluated in parallel by the two techniques described above.

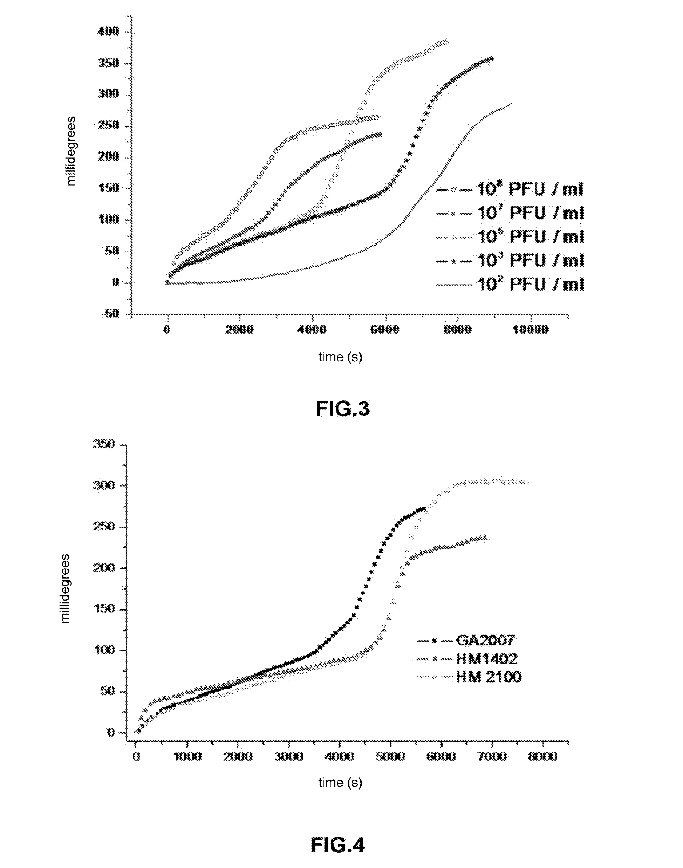

Figure 3 shows a graphic representation over time of the normalised measurements of the resonance angle for the series of dilutions described above. The measurements shown were those taken during the incubation phase of these solutions, wherein the increase in the resonance angle caused by phage activity took place.

As can be seen in Figure 3, the gradient of the calibration curves shown is more pronounced for those solutions with a higher bacteriophage concentration, the time for detection of the change in resonance angle being less as the concentration was higher.

The experiments performed demonstrated that the detection limit of the method is 10² PFU/ml (Plaque Forming Units), an increase in the resonance angle occurring in a time no greater than 2 hours in all cases.

In Figure 4, normalised measurements of the resonance angle of three samples of reference waste water analysed, GA2007, HM1402 and HM2100, are shown. The measurements shown are those taken during the incubation phase of the samples, wherein the increase in the resonance angle due to lysis occur.

As can be seen in Figure 4, the gradient of the normalised curve is greater for the reference sample GA2007, which indicates that the bacteriophage concentration in this sample is higher than that of the other samples, the change in the resonance angle being detected in a shorter time.

The calculation of the bacteriophage concentration of each of the samples of Figure 4 is determined according to the time elapsed for the detection of the change in the resonance angle by means of the corresponding calibration curve, which correlates the time of detection of the change with the bacteriophage concentration.

Surprisingly, the present invention provides a system and method for detecting bacteriophages that is very sensitive and also very reliable, as it has been demonstrated that the results obtained with the SPR method show a high correlation with the results obtained by means of the traditional double agar layer assay.

In the described embodiment, attachment of bacteria 2 to the surface of the chip 1 was carried out by the adsorption of anavidin solution, whereto the previously biotinylated bacteria 2 are bound. Nevertheless, as was mentioned in the description of the invention, the attachment of bacteria can be performed by any other equivalent technique for immobilising bacteria 2 to the conductive material surface 1 a of the sensor chip 1.

For example, it has been observed that the immobilisation of biotinylated bacteria by adsorption of a CaptAvidin solution significantly improves the sensitivity of the bacteriophage detection process. In particular, an increase in sensitivity of up to 8% in the detection of somatic coliphages capable of infecting bacterial strains of *Escherichia coli* has been observed.

Immobilisation using CaptAvidin has the added advantage that it enables the dissociation of the complex formed with the biotin of the bacteria and, therefore, the regeneration of the detection surface of the sensor. This result is especially advantageous for industrial on-line implementation of the claimed device and detection process.

Furthermore, the embodiment described relates to the detection of somatic coliphage bacteriophages that infect *Escherichia coli.* Nevertheless, as has been mentioned, the present invention can be applied to the detection and/or quantification of any type of bacteriophage provided that a bacterial host strain susceptible to infection by this bacteriophage is available.

## Claims

1. Process for detecting and/or quantifying bacteriophages capable of infecting a predetermined bacterial host strain, comprising the stages of:
a) attaching bacteria from at least one of said host strains to the conductive material surface (1 a) in a microelectronic optical sensor device (1) based on the surface plasmon resonance technique,
b) exposing the bacteria (2) attached to said surface to a solution of material to be analysed that may contain bacteriophages,
c) incubating the solution from stage b) in predetermined conditions with the aim that, if the bacteria (2) attached to the conductive material surface (1 a) are infected by bacteriophages, these bacteria (2) will be lysed,
d) measuring the resonance angle of incident light on the sensor (1) while carrying out the incubation of stage c) with the aim of detecting the change in the resonance angle caused by bacterial lysis.

2. Process according to claim 1, further comprising the stage of:
- determining the bacteriophage concentration of the solution according to the time elapsed before detecting the change in the resonance angle using the corresponding calibration curve, which correlates the detection time with the bacteriophage concentration.

3. Process according to claim 1, where the bacterial attachment of stage a) comprises the previous adsorption to said conductive material surface (1 a) of chemical or biological material for immobilising bacteria (2) to the sensor (1).

4. Process according to claim 3, where said biological material includes avidin or a protein derived from avidin.

5. Process according to claim 4, where said protein derived from avidin is CaptAvidin.

6. Process according to any of the preceding claims, wherein said bacteria are biotinylated bacteria (2).

7. Process according to any of the preceding claims, wherein said bacteriophages are somatic coliphages or bacteriophages capable of infecting strains of *Escherichia coli.*

8. Process according to any of the preceding claims 1 to 6, wherein said bacteriophages are bacteriophages capable of infecting strains of lactic bacteria.

9. Process according to any of the preceding claims, wherein the solution of material to be analysed comes from a sample of liquid material or material soluble in a liquid.

10. Process according to claim 9, wherein the solution of material to be analysed comes from a water sample.

11. Microelectronic optical sensor device for detecting and/or quantifying bacteriophages according to the method of any of claims 1 to 10, that being based on the surface plasmon resonance technique, is **characterised in that** it comprises bacteria (2) attached to the conductive material surface (1 a) of the sensor (1), these bacteria (2) belonging to at least one predetermined host strain, selected to be infected by bacteriophages that are specific for said strain.

12. Device according to claim 11, wherein said conductive material (1 a) is gold.

13. System for detecting and/or quantifying bacteriophages using the device according to any of claims 11 to 12, comprising:
- a microelectronic optical sensor device (1) based on the surface plasmon resonance technique,
- bacteria (2) of said host strain, attached to the conductive material surface (1 a) of said device (1), and
- means of processing and control for detecting the change in resonance angle of light incident on the sensor (1), said change being caused by the effect of lysis when said bacteria (2) are infected and lysed by said bacteriophages.

14. Use of a microelectronic optical sensor device according to any of claims 11 to 12, for detecting and/or quantifying bacteriophages capable of infecting a predetermined bacterial host strain by surface plasmon resonance.
